# EUROPEAN PATENT APPLICATION

(11) **EP 2 050 741 A1**
(43) Date of publication of application: **22.04.2009**
(21) Application number: 07791047.9
(22) Date of filing: 20.07.2007
(51) Int. Cl.: C07D 295/08, A61K 31/495, A61P 3/10, A61P 9/00, A61P 9/04, A61P 9/06, A61P 9/10, A61P 9/12, A61P 21/00, A61P 25/00

(54) **CRYSTALLINE POLYMORPHISM OF 5-METHYL-2-(PIPERAZIN-1-YL)BENZENESULFONIC ACID**

(30) Priority: 21.07.2006 JP 2006200072
(71) Applicant: Mitsubishi Tanabe Pharma Corporation, Chuo-ku Osaka-shi Osaka 541-8505 (JP)
(72) Inventor: OONO, Chika, Chuo-ku, Osaka-shi, Osaka 541-8505 (JP); WAKASUGI, Takeshi, Chuo-ku, Osaka-shi, Osaka 541-8505 (JP); MASUDA, Katsuhiko, Chuo-ku, Osaka-shi, Osaka 541-8505 (JP)
(74) Representative: HOFFMANN EITLE
(86) International application number: PCT/JP2007/064309
(87) International publication number: WO 2008/010566

(57) **Abstract**

The present invention provides novel crystals of 5-methyl-2-(piperazin-1-yl)benzenesulfonic anhydrate and monohydrate.
The present invention provides type I crystal and type II crystal of 5-methyl-2-(piperazin-1-yl)benzenesulfonic anhydrate, which show particular diffraction peaks in powder X-ray diffraction pattern, as well as type II crystal and type III crystal of 5-methyl-2-(piperazin-1-yl)benzenesulfonic monohydrate, which show particular diffraction peaks in powder X-ray diffraction pattern.

## Description

### Technical Field

The present invention relates to a novel crystalline polymorph of 5-methyl-2-(piperazin-1-yl)benzenesulfonic acid and a pharmaceutical agent comprising same as an active ingredient.

### Background Art

An ability of a substance to crystallize into two or more kinds of crystal structures is known as polymorphism, and individual crystal forms are called crystalline polymorphs. When crystalline polymorphism is different, even if the same compound sometimes shows completely different properties such as preservation stability, solubility and the like. When the compound is used as a pharmaceutical product, such difference in the properties may influence the action effect. Thus, it is useful to study whether a compound to be used as a pharmaceutical product has crystalline polymorphism.

It is known that an aminobenzenesulfonic acid derivative represented by the following formula (I)

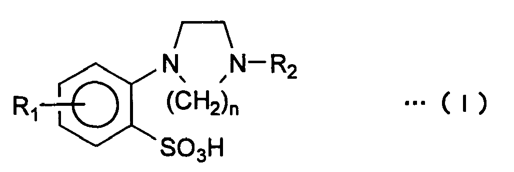

wherein R₁ is a hydrogen atom, a C₁-C₆ alkyl group, a C₃-C₇ cycloalkyl group, a C₁-C₄ halogenated alkyl group, a halogen atom, or a C₆-C₁₂ aryl group; R₂ is a hydrogen atom, a C₁-C₆ alkyl group or a C₇-C₁₂ aralkyl group optionally having one or more substituents selected from the group consisting of a cyano group, a nitro group, a C₁-C₆ alkoxy group, a halogen atom, a C₁-C₆ alkyl group and an amino group; and n is an integer of 1 to 4,
or a salt thereof or a hydrate or solvate thereof suppresses excessive accumulation of intracellular calcium ions in cardiac muscle and blood vessel smooth muscle (patent reference 1). Of such compounds, 5-methyl-2-(piperazin-1-yl)benzenesulfonic acid represented by the following formula (II)

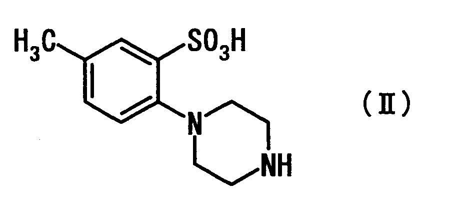

(indicated as 2-(1-piperazinyl)-5-methylbenzenesulfonic acid in patent reference 1; a product of compound No. 12 disclosed in Example of patent reference 1; hereinafter sometimes to be referred to as "compound A") remarkably suppresses excessive influx of calcium ions into cardiomyocytes and shows high safety. Accordingly, it is expected to be extremely useful as an active ingredient of therapeutic agents and/or prophylactic agents for cardiac diseases.

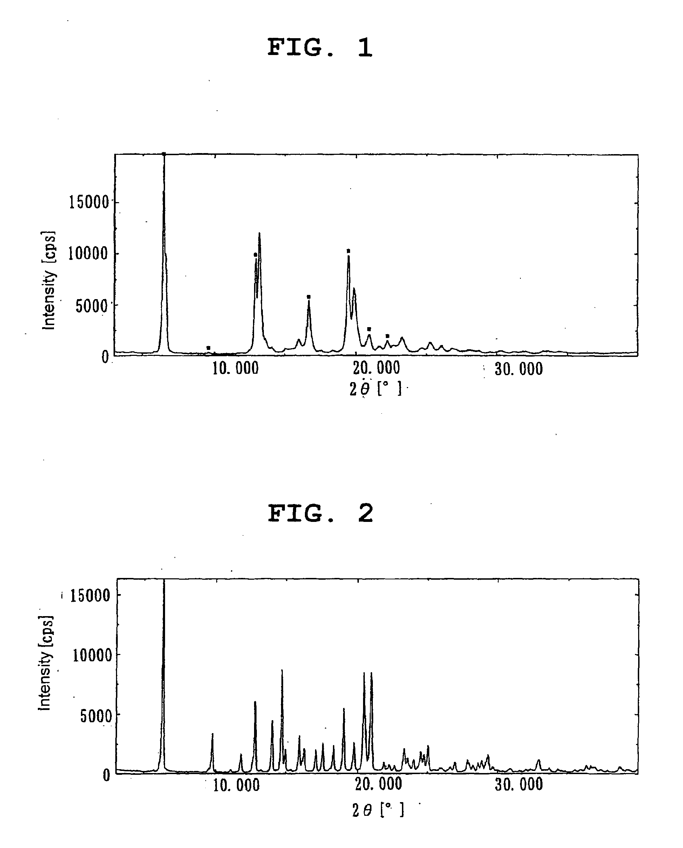

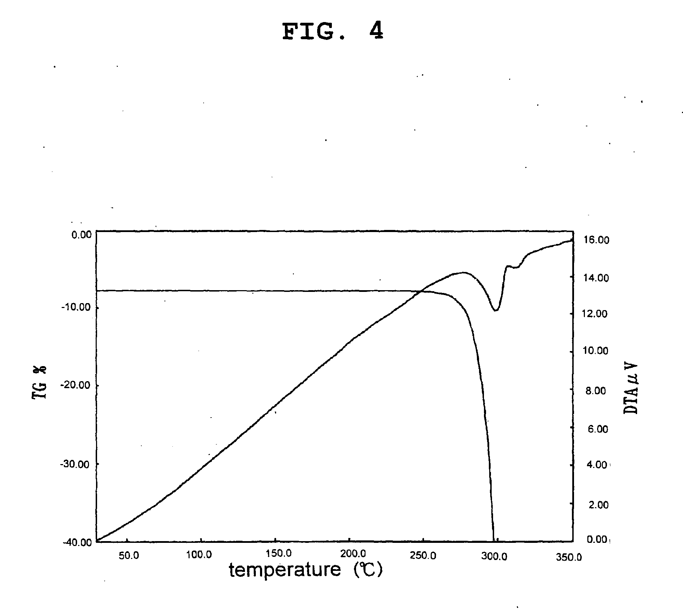

As regards compound A, patent reference 2 describes compound A can be obtained as an anhydride according to the method described in Example 1 of patent reference 1. In addition, Figs. 2 and 4 of patent reference 2 each describe differential thermal analysis and powder X-ray diffraction of compound A anhydrate. However, they do not describe whether compound A anhydrate has crystalline polymorphism and, even if it does, what kind of crystalline polymorphism it is.

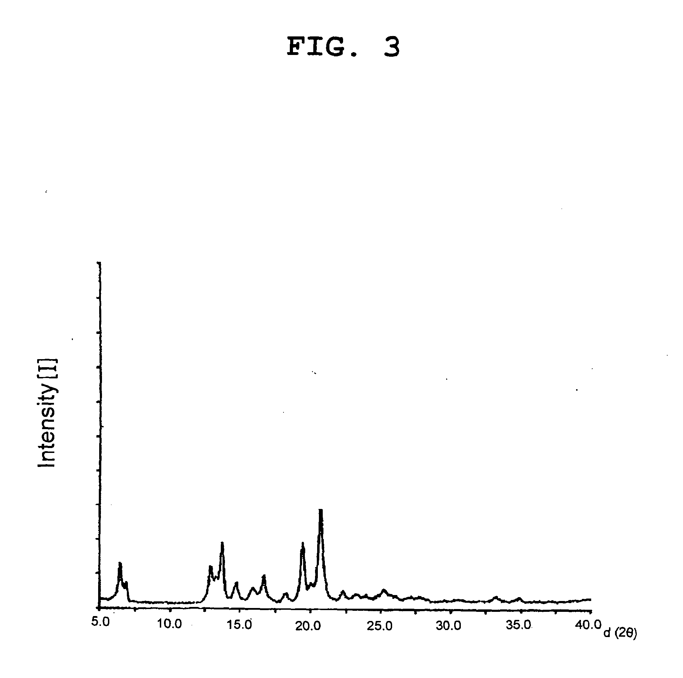

Moreover, patent reference 2 describes monohydrate of compound A. Fig. 1 and Fig. 3 of patent reference 2 each describe differential thermal analysis and powder X-ray diffraction of compound A monohydrate. However, they do not describe whether compound A monohydrate has crystalline polymorphism and, even if it does, what kind of crystalline polymorphism it is.
patent reference 1: JP-A-3-7263, EP-A-390654
patent reference 2: JP-A-9-221479, EP-A-0779283

### Disclosure of the Invention

### Problems to be Solved by the Invention

An object of the present invention is to provide a novel crystal of 5-methyl-2-(piperazin-1-yl)benzenesulfonic acid anhydrate (hereinafter one of the two kinds of crystal forms is referred to as type-I anhydrate crystal and the other is referred to as type-II anhydrate crystal in the present specification).

An object of the present invention is to provide a novel crystal of 5-methyl-2-(piperazin-1-yl)benzenesulfonic acid monohydrate (hereinafter one of the two kinds of crystal forms is referred to as type-II monohydrate crystal and the other is referred to as type-III monohydrate crystal in the present specification).

### Means of Solving the Problems

The present inventors have conducted intensive studies in an attempt to solve the above-mentioned problems and found type-I and -II crystals of 5-methyl-2-(piperazin-1-yl)benzenesulfonic acid anhydrate, and further, type-II and - III crystals of 5-methyl-2-(piperazin-1-yl)benzenesulfonic acid monohydrate, which resulted in the completion of the present invention.

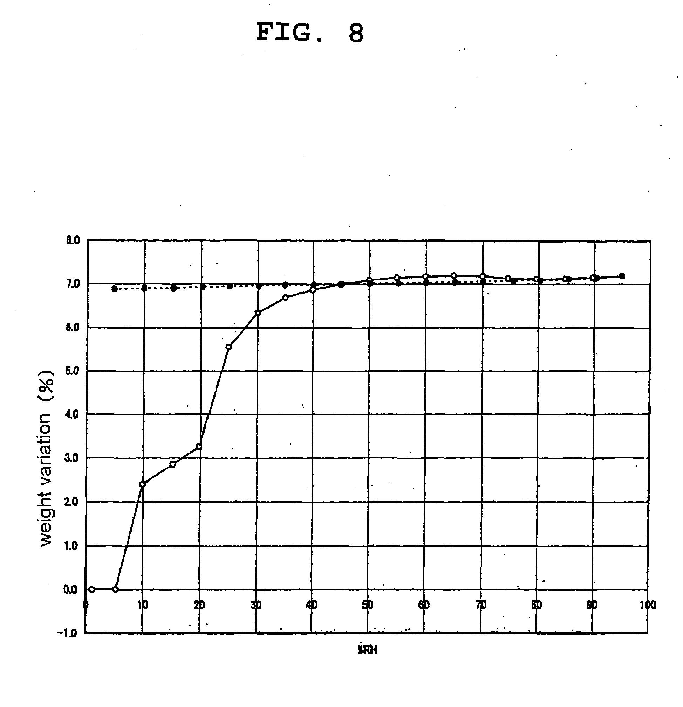

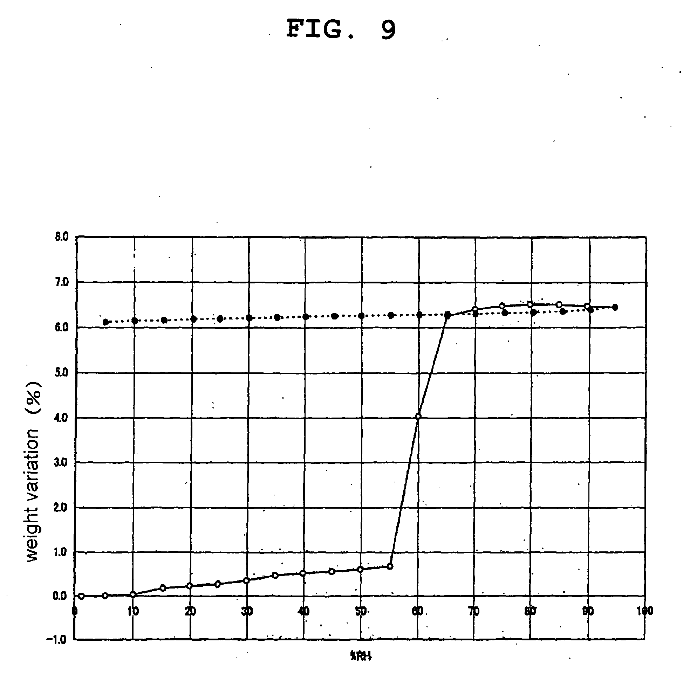

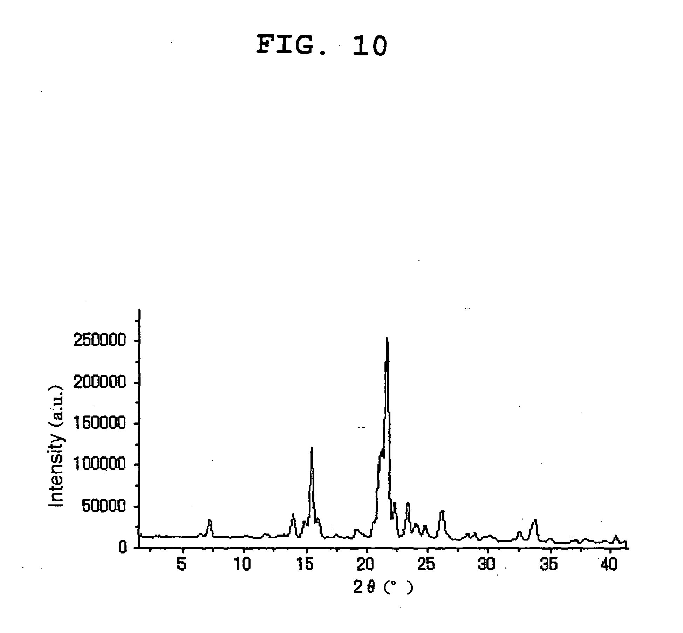

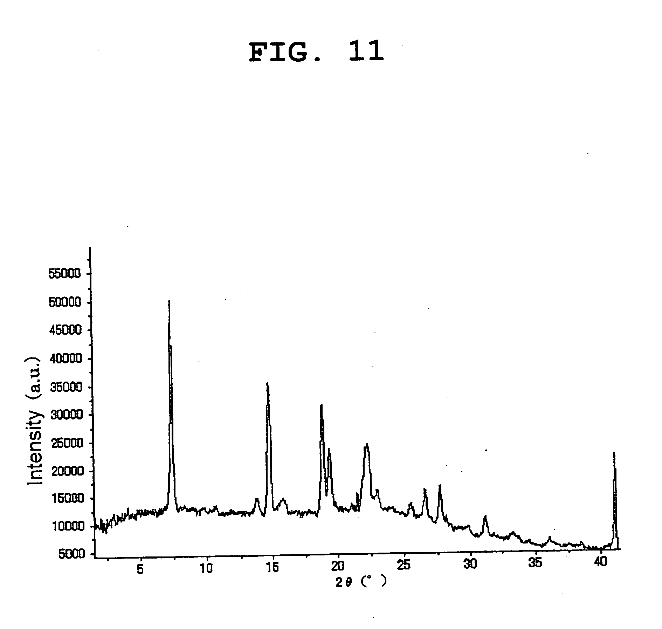

Accordingly, the present invention relates to the following.
(1) 5-Methyl-2-(piperazin-1-yl)benzenesulfonic acid anhydrate characterized by the following (a) and (b):
   (a) showing a diffraction peak at at least one Bragg angle (2θ) selected from 6.4°, 13.0°, 16.7°, 19.5° and 19.8° (each ±0.2°) in a powder X-ray diffraction pattern,
   (b) showing no diffraction peak at any of Bragg angles (2θ) of 14.0°, 14.7° and 19.1° (each ±0.2°) in a powder X-ray diffraction pattern.
(2) The 5-methyl-2-(piperazin-1-yl)benzenesulfonic acid anhydrate of the aforementioned (1), showing diffraction peaks at Bragg angles (2θ) 16.7° and 19.5° (each ±0.2°) in a powder X-ray diffraction pattern.
(3) The 5-methyl-2-(piperazin-1-yl)benzenesulfonic acid anhydrate of the aforementioned (1), showing diffraction peaks at Bragg angles (2θ) of 6.4°, 16.7° and 19.5° (each ±0.2°) in a powder X-ray diffraction pattern.
(4) The 5-methyl-2-(piperazin-1-yl)benzenesulfonic acid anhydrate of the aforementioned (1), showing diffraction peaks at Bragg angles (2θ) of 6.4°, 13.0°, 16.7° and 19.5° (each ±0.2°) in a powder X-ray diffraction pattern.
(5) The 5-methyl-2-(piperazin-1-yl)benzenesulfonic acid anhydrate of the aforementioned (1), showing diffraction peaks at Bragg angles (2θ) of 6.4°, 13.0°, 16.7°, 19.5° and 19.8° (each ±0.2°) in a powder X-ray diffraction pattern.
(6) 5-Methyl-2-(piperazin-1-yl)benzenesulfonic acid anhydrate having substantially the same powder X-ray diffraction pattern as that showing Bragg angles (2θ) in the powder X-ray diffraction pattern of Fig. 1.
(7) 5-Methyl-2-(piperazin-1-yl)benzenesulfonic acid anhydrate showing substantially the same moisture sorption isotherms as that of Fig. 8.
(8) The 5-methyl-2-(piperazin-1-yl)benzenesulfonic acid anhydrate of any of the aforementioned (1) to (6), which shows substantially the same moisture sorption isothermsas that of Fig. 8.
(9) 5-Methyl-2-(piperazin-1-yl)benzenesulfonic acid anhydrate showing moisture sorptionat relative humidity 50% of not less than about 6%.
(10) The 5-methyl-2-(piperazin-1-yl)benzenesulfonic acid anhydrate of any of the aforementioned (1) to (6), showing moisture sorption at relative humidity 50% of not less than about 6%.
(11) 5-Methyl-2-(piperazin-1-yl)benzenesulfonic acid anhydrate characterized by the following (c) and (d):
   (c) showing a diffraction peak at at least one Bragg angle (2θ) selected from 6.3°, 9.8°, 12.8°, 14.0°, 14.7° and 19.1° (each ±0.2°) in a powder X-ray diffraction pattern,
   (d) showing no diffraction peak at any of Bragg angles (2θ) of 16.7° and 19.5° (each ±0.2°) in a powder X-ray diffraction pattern.
(12) The 5-methyl-2-(piperazin-1-yl)benzenesulfonic acid anhydrate of the aforementioned (11), showing diffraction peaks at Bragg angles (2θ) of 14.0° and 14.7° (each ±0.2°) in a powder X-ray diffraction pattern.
(13) The 5-methyl-2-(piperazin-1-yl)benzenesulfonic acid anhydrate of the aforementioned (11), showing diffraction peaks at Bragg angles (2θ) of 14.0°, 14.7° and 19.1° (each ±0.2°) in a powder X-ray diffraction pattern.
(14) The 5-methyl-2-(piperazin-1-yl)benzenesulfonic acid anhydrate of the aforementioned (11), showing diffraction peaks at Bragg angles (2θ) of 6.3°, 14.0°, 14.7° and 19.1° (each ±0.2°) in a powder X-ray diffraction pattern.
(15) The 5-methyl-2-(piperazin-1-yl)benzenesulfonic acid anhydrate of the aforementioned (11), showing diffraction peaks at Bragg angles (2θ) of 6.3°, 9.8°, 14.0°, 14.7° and 19.1° (each ±0.2°) in a powder X-ray diffraction pattern.
(16) 5-Methyl-2-(piperazin-1-yl)benzenesulfonic acid anhydrate having substantially the same powder X-ray diffraction pattern as that showing Bragg angles (2θ) in the powder X-ray diffraction pattern of Fig. 2.
(17) 5-Methyl-2-(piperazin-1-yl)benzenesulfonic acid anhydrate showing substantially the same moisture sorption isotherms as that of Fig. 9.
(18) The 5-methyl-2-(piperazin-1-yl)benzenesulfonic acid anhydrate of any of the aforementioned (11) to (16), showing substantially the same moisture sorption isotherms as that of Fig. 9.
(19) 5-Methyl-2-(piperazin-1-yl)benzenesulfonic acid anhydrate showing moisture sorption at relative humidity 50% of not more than about 2%.
(20) The 5-methyl-2-(piperazin-1-yl)benzenesulfonic acid anhydrate of any of the aforementioned (11) to (20), showing moisture sorption at relative humidity 50% of not more than about 2%.
(21) 5-Methyl-2-(piperazin-1-yl)benzenesulfonic acid monohydrate characterized by the following (e) and (f):
   (e) showing a diffraction peak at at least one Bragg angle (2θ) selected from 7.3°, 14.0°, 15.5°, 19.1°, 21.7° and 26.2° (each ±0.2°) in a powder X-ray diffraction pattern,
   (f) showing no diffraction peak at any of Bragg angles (2θ) of 13.3°, 25.4° and 27.6° (each ±0.2°) in a powder X-ray diffraction pattern.
(22) 5-Methyl-2-(piperazin-1-yl)benzenesulfonic acid monohydrate showing substantially the same powder X-ray diffraction pattern as that showing Bragg angles (2θ) in the powder X-ray diffraction pattern of Fig. 10.
(23) 5-Methyl-2-(piperazin-1-yl)benzenesulfonic acid monohydrate characterized by the following (g) and (h):
   (g) showing a diffraction peak at at least one Bragg angle (2θ) selected from 7.4°, 14.7°, 18.9°, 19.4° and 22.3° (each ±0.2°) in a powder X-ray diffraction pattern,
   (h) showing no diffraction peak at any of Bragg angles (2θ) of 13.3°, 13.7°, 15.6°, 25.4° and 27.6° (each ±0.2°) in a powder X-ray diffraction pattern.
(24) 5-Methyl-2-(piperazin-1-yl)benzenesulfonic acid monohydrate having substantially the same powder X-ray diffraction pattern as that showing Bragg angles (2θ) in the powder X-ray diffraction pattern of Fig. 11.
(25) A drug substance consisting of the 5-methyl-2-(piperazin-1-yl)benzenesulfonic acid anhydrate of any of the aforementioned (1) to (20).
(26) A drug substance consisting of the 5-methyl-2-(piperazin-1-yl)benzenesulfonic acid monohydrate of any of the aforementioned (21) to (24).
(27) A pharmaceutical composition comprising the 5-methyl-2-(piperazin-1-yl)benzenesulfonic acid anhydrate of any of the aforementioned (1) to (20) as an active ingredient.
(28) A pharmaceutical composition comprising the 5-methyl-2-(piperazin-1-yl)benzenesulfonic acid monohydrate of any of the aforementioned (21) to (24) as an active ingredient.
(29) The pharmaceutical composition of the aforementioned (27) or (28), which is used for the prophylaxis and/or treatment of an ischemic cardiac disease or an ischemic circulatory disorder.
(30) The pharmaceutical composition of the aforementioned (27) or (28), which is used for the prophylaxis and/or treatment of muscle infarction, angina pectoris, cardiac failure, hypertension, arrhythmia, cardiomyopathy, diastolic disorder, nerve system disorder, cerebrovascular disorder, ischemic cerebrovascular disorder, or cardiac failure or arrhythmia in ischemic cardiac disease derived from diabetes.
(31) The pharmaceutical composition of the aforementioned (27) or (28), which is used for the prophylaxis and/or treatment of a circulatory disorder by administration to a patient undergoing percutaneous coronary intervention.
(32) A method of producing a type-I crystal of the 5-methyl-2-(piperazin-1-yl)benzenesulfonic acid anhydrate of any of the aforementioned (1) to (10), which comprises crystallizing or washing, in a suspension state, 5-methyl-2-(piperazin-1-yl)benzenesulfonic acid solvate using a solvent containing water, and drying the obtained crystal.
(33) A method of producing a type-II crystal of the 5-methyl-2-(piperazin-1-yl)benzenesulfonic acid anhydrate of any of the aforementioned (11) to (20), which comprises crystallizing or washing, in a suspension state, 5-methyl-2-(piperazin-1-yl)benzenesulfonic acid solvate using a solvent free of water.

### Effect of the Invention

According to the present invention, type-I anhydrate crystal and type-II anhydrate crystal, which are novel crystalline polymorphs, of 5-methyl-2-(piperazin-1-yl)benzenesulfonic acid anhydrate can be provided.

According to the present invention, type-II monohydrate crystal and type-III monohydrate crystal, which are novel crystalline polymorphs, of 5-methyl-2-(piperazin-1-yl)benzenesulfonic acid monohydrate can be provided.

### Brief Description of the Drawings

Fig. 1 shows a powder X-ray diffraction pattern of type-I anhydrate crystal.
Fig. 2 shows a powder X-ray diffraction pattern of type-II anhydrate crystal.
Fig. 3 shows a powder X-ray diffraction pattern of the anhydride crystal described in Example 1 of JP-A-3-7263.
Fig. 4 shows thermal analysis curve of type-I anhydrate crystal.
Fig. 5 shows thermal analysis curve of type-II anhydrate crystal.
Fig. 6 shows infrared spectrum of type-I anhydrate crystal.
Fig. 7 shows infrared spectrum of type-II anhydrate crystal.
Fig. 8 shows the moisture sorption isotherms of type-I anhydrate crystal at each relative humidity, wherein a solid line shows increase in the relative humidity (5%RH → 95%RH), and a dotted line shows decrease in the relative humidity (95%RH → 5%RH) .
Fig. 9 shows the moisture sorption isotherms of type-II anhydrate crystal at each relative humidity, wherein a solid line shows increase in the relative humidity (5%RH → 95%RH), and a dotted line shows decrease in the relative humidity (95%RH → 5%RH).
Fig. 10 shows a powder X-ray diffraction pattern of type-II monohydrate crystal.
Fig. 11 shows a powder X-ray diffraction pattern of type-III monohydrate crystal.
Fig. 12 shows thermal analysis curve of type-II monohydrate crystal.
Fig. 13 shows thermal analysis curve of type-III monohydrate crystal.

### Best Mode for Carrying Out the Invention

The present invention is explained in detail in the following.

The type-I anhydrate crystal and type-II anhydrate crystal of the present invention are crystalline polymorphs of 5-methyl-2-(piperazin-1-yl)benzenesulfonic acid anhydrate represented by the aforementioned formula (II), and they have different physical characteristics.

The type-I anhydrate crystal of the present invention has the following physical characteristics absent in type-II anhydrate crystal and specific to type-I anhydrate crystal. Accordingly, it is characterized by the following (a) and (b).
(a) It shows a diffraction peak at at least one Bragg angle (2θ) selected from 6.4°, 13.0°, 16.7°, 19.5° and 19.8° (each ±0.2°) in a powder X-ray diffraction pattern.
(b) It does not show a diffraction peak at any of Bragg angles (2θ) 14.0°, 14.7° and 19.1° (each ±0.2°) in a powder X-ray diffraction pattern.

Moreover, the type-I anhydrate crystal of the present invention preferably shows a diffraction peak at Bragg angles (2θ) 16.7° and 19.5° (each ±0.2°) in a powder X-ray diffraction pattern.

The type-II anhydrate crystal of the present invention has the following physical characteristics absent in type-I anhydrate crystal and specific to type-I anhydrate crystal. Accordingly, it is characterized by the following (c) and (d). (c) It shows a diffraction peak at at least one Bragg angle (2θ) selected from 6.3°, 9.8°, 12.8°, 14.0°, 14.7° and 19.1° (each ±0.2°) in a powder X-ray diffraction pattern.
(d) It does not show a diffraction peak at any of Bragg angles (2θ) 16.7° and 19.5° (each ±0.2°) in a powder X-ray diffraction pattern.

Moreover, the type-II anhydrate crystal of the present invention preferably shows a diffraction peak at Bragg angles (2θ) 14.0°, 14.7° and 19.1° (each ±0.2°) in a powder X-ray diffraction pattern.

The type-II monohydrate crystal of the present invention has the following physical characteristics absent in type-III monohydrate crystal and specific to type-II monohydrate crystal. Accordingly, it is characterized by the following (e) and (f).
(e) It shows a diffraction peak at at least one Bragg angle (2θ) selected from 7.3°, 14.0°, 15.5°, 19.1°, 21.7° and 26.2° (each ±0.2°) in a powder X-ray diffraction pattern.
(f) It does not show a diffraction peak at any of Bragg angles (2θ) 13.3°, 25.4° and 27.6° (each ±0.2°) in a powder X-ray diffraction pattern.

Moreover, the type-II monohydrate crystal of the present invention preferably shows a diffraction peak at Bragg angles (2θ) 14.0°, 15.5° and 26.2° (each ±0.2°) in a powder X-ray diffraction pattern.

The type-III monohydrate crystal of the present invention has the following physical characteristics absent in type-II monohydrate crystal and specific to type-III monohydrate crystal. Accordingly, it is characterized by the following (g) and (h).
(g) It shows a diffraction peak at at least one Bragg angle (2θ) selected from 7.4°, 14.7°, 18.9°, 19.4° and 22.3° (each ±0.2°) in a powder X-ray diffraction pattern.
(h) It does not show a diffraction peak at any of Bragg angles (2θ) 13.3°, 13.7°, 15.6°, 25.4° and 27.6° (each ±0.2°) in a powder X-ray diffraction pattern.

Moreover, the type-III monohydrate crystal of the present invention preferably shows a diffraction peak at Bragg angles (2θ) 14.7°, 18.9° and 22.3° (each ±0.2°) in a powder X-ray diffraction pattern.

The powder X-ray diffraction pattern in the present invention refers to an X-ray diffraction pattern measured using, for example, RINT2500 type powder X-ray apparatus (Rigaku Corporation) or powder X-ray diffractometer (manufactured by Bruker) under the following conditions. Strictly speaking, however, the apparatus and method are not limited to them.
X-ray source : Cu
filter : Ni
tube voltage : 40 kV
tube current : 300 mA
divergence slit: 1/2°
scattering slit: 1/2°
receiving slit: 0.15 mm
scanning range : 3 - 40° 2θ
angular step : 0.02°
sampling time: 1.00 sec
sample rotation speed : 60 rpm
scan speed: 0.02°/sec
5-Methyl-2-(piperazin-1-yl)benzenesulfonic acid anhydrate crystals showing a generally similar data pattern by the analysis using such apparatus are encompassed in the crystal of the present invention. For example, type-I anhydrate crystal containing type-II anhydrate crystal in an amount undetectable by general measurement methods should be considered to be the type-I anhydrate crystal of the present invention, and type-II anhydrate crystal containing type-I anhydrate crystal in an amount undetectable by general measurement methods should be considered to be the type-II anhydrate crystal of the present invention.

Moreover, 5-methyl-2-(piperazin-1-yl)benzenesulfonic acid monohydrate crystals showing a generally similar data pattern by analysis are encompassed in the crystal of the present invention. For example, type-II monohydrate crystal containing type-III monohydrate crystal in an amount undetectable by general measurement methods should be considered to be the type-II monohydrate crystal of the present invention, and type-III monohydrate crystal containing type-II monohydrate crystal in an amount undetectable by general measurement methods should be considered to be the type-III monohydrate crystal of the present invention.

When the identity of crystals is to be determined by powder X-ray diffraction pattern, the presence or absence of each diffraction peak at Bragg angles (2θ) and the overall pattern are important, and the relative intensity may vary somewhat depending on the direction of crystal growth, particle size, and measurement conditions. Thus, crystals showing a generally similar powder X-ray diffraction pattern as that of the crystal of the present invention should be considered to be encompassed in the crystal of the present invention, and particularly, crystals showing a diffraction peak in the same plane indeces are encompassed in the present invention.

The type-I anhydrate crystal of the present invention characteristically shows moisture sorption at relative humidity 50% of not less than about 6%. In addition, the type-II anhydrate crystal of the present invention characteristically shows moisture sorption at relative humidity 50% of not more than about 2%.

In the present invention, when moisture sorption at each relative humidity becomes not more than 0.03% in 5 min, or when 3 hours have passed at each relative humidity, the range of relative humidity being from 0% to 95%, the humidity is increased or decreased at 5% intervals, and the moisture sorption isotherms at each relative humidity is obtained gravimetrically by measuring the mass change of a sample.

For example, when the anhydride of the present invention shows moisture sorption at relative humidity 50% based on the moisture sorption isotherms of not less than about 6%, it is determined to be the type-I anhydrate crystal of the present invention, and when the anhydride shows moisture sorption at relative humidity 50% of not more than about 2%, it is determined to be the type-II anhydrate crystal of the present invention.

The above-mentioned moisture sorption can be measured using, for example, SGA-100 type water vapor sorption apparatus (manufactured by VTI) as shown in the Examples. While the error in the relative humidity values depends on the performance of the apparatus, it is about ±1% using this apparatus.

The "moisture sorption" in the present invention refers to the values calculated by the following equation:

moisture sorption (%)=((RM-DM)/DM))*100
wherein
DM: sample weight (mg) after sufficient drying at relative humidity 0%
RM: sample weight (mg) in the equilibrium state at various relative humidities
The type-II anhydrate crystal of the present invention advantageously shows poor hygroscopicity and is crystallographically stable as compared to 5-methyl-2-(piperazin-1-yl)benzenesulfonic acid anhydrate obtained according to the method described in Example 1 of the aforementioned patent reference 1 (hereinafter sometimes to be referred to as "known product 1"). Therefore, it is useful as an active ingredient? of a pharmaceutical product.

By way of the type-I anhydrate crystal of the present invention, 5-methyl-2-(piperazin-1-yl)benzenesulfonic acid showing less moisture sorption at everyday relative humidity of 40 - 60% can be advantageously provided, and the anhydrate crystal shows high operability.

Both the type-II monohydrate crystal and type-III monohydrate crystal of the present invention did not show noticeable endothermic and exothermic peaks up to near 80°C by thermal analysis, and were confirmed to be stable crystals. Accordingly, the novel crystal of the present invention can be supplied stably as an active ingredient drug of pharmaceutical products.

While the production method of the crystal of the present invention is not particularly limited, it includes, for example, as shown in the following Examples, a step of drying 5-methyl-2-(piperazin-1-yl)benzenesulfonic acid monohydrate obtained by the method described in Example 1 of patent reference 2, a step of dissolving the substance in a solvent and crystallizing the same, or a step of concentrating a solution of the substance and crystallizing the same. Preferably, the method includes a step of stirring a solid substance in a suspension state in a solvent.

Examples of the above-mentioned solvent include various solvents acceptable for the production of general pharmaceutical products such as N,N-dimethylformamide, ethyl acetate, chloroform, cyclohexane, ethanol, methanol, acetone, acetonitrile, water etc., and a mixed solvent thereof.
The production method of type-I anhydrate crystal is more preferably a method including crystallizing or washing, in a suspension state, 5-methyl-2-(piperazin-1-yl)benzenesulfonic acid monohydrate using a solvent containing water, and drying the obtained crystal.
In the present invention, "washing, in a suspension state" means an operation to agitate a solid substance in the state of a suspension in a solvent.
In the present invention, "drying" means an operation to remove a solvent adsorbed onto a solid substance by subjecting the solid substance to heating and/or reducing pressure and/or blowing a stream and the like. Drying is preferably performed by heating (not less than 80°C) or under reduced pressure (for example, not more than 1 mmHg).
The production method of type-II anhydrate crystal is more preferably a method including crystallizing or washing, in a suspension state, 5-methyl-2-(piperazin-1-yl)benzenesulfonic acid monohydrate using a solvent free of water.
In the present invention, the "solvent free of water" means the following organic solvents having a water content of not more than 0.2%, preferably, N,N-dimethylformamide and the like.

Utilizing the crystal of the present invention, a mixture of type-I anhydrate crystal and type-II anhydrate crystal at any ratio can be obtained. For example, type-I anhydrate crystal and type-II anhydrate crystal obtained by the above-mentioned production methods are mixed at a desired ratio to give a mixture containing type-I anhydrate crystal and type-II anhydrate crystal at any ratio.
When the crystal of the present invention is administered as a pharmaceutical product, it is orally or parenterally administered to human by a conventional method. Examples of the dosage form for oral administration include granule, fine granule, powder, tablet, hard capsule, soft capsule, syrup, emulsion, suspension, liquid and the like. Examples of the dosage form for parenteral administration include injection, suppository, transdermal agent and the like.

A pharmaceutical composition containing the crystal of the present invention as an active ingredient contains, in the above-mentioned dosage form, a solid or liquid carrier or conventional additive for pharmaceutical agents such as excipient, stabilizer, lubricant, sweetening agent, preservative, suspending agent and the like, where the content ratio of the prophylactic and/or therapeutic active ingredient relative to the carrier component is preferably within the range of 1 wt%-90 wt%.

The crystal of the present invention has an action to suppress excessive accumulation of intracellular calcium ions in cardiac muscle or blood vessel smooth muscle, an action to inhibit sodium/calcium exchange system, and an action to suppress excessive accumulation of intracellular sodium ions. Accordingly, a pharmaceutical agent containing the crystal of the present invention as an active ingredient is useful for the treatment and/or prophylaxis of circulatory diseases and the like caused by excessive accumulation of intracellular calcium ions and/or excessive accumulation of intracellular sodium ions.

In addition, a pharmaceutical agent containing the crystal of the present invention as an active ingredient is effective for the treatment and/or prophylaxis of ischemic cardiac diseases or ischemic circulatory disorders, and preferably, effective for the treatment and/or prophylaxis of myocardial infarction, angina pectoris, cardiac failure, hypertension, arrhythmia, cardiomyopathy, diastolic disorder, nerve system disorder, cerebrovascular disorder, ischemic cerebrovascular disorder, or cardiac failure or arrhythmia in ischemic cardiac diseases derived from diabetes.

A pharmaceutical agent containing the crystal of the present invention as an active ingredient is used for the prophylaxis and/or treatment of circulatory disorders. It is effective as a pharmaceutical agent to be administered to patients undergoing percutaneous coronary intervention, and also effective for the treatment of myocardial infarction and the like in such patients.

The ischemic circulatory disorder in the present invention refers to a disorder in the circulatory organ, which is caused by blockage of the blood vessel due to some reason such as formation of thrombus and the like, and specifically means, for example, myocardial infarction or angina pectoris. The type of myocardial infarction, for which the present invention is effective, is ST segment elevation myocardial infarction (STEMI).

The dose of the crystal of the present invention to be used as an active ingredient can be appropriately determined for each active ingredient in consideration of the object of prophylaxis or treatment, the kind of disease to be prevented or treated, symptom, body weight, age, sex and the like of patients. In general, about 0.001 mg - 100 mg can be administered per day to an adult by parenteral administration, and about 0.01 mg - 1000 mg can be administered by oral administration. Such dose is desirably administered in one to several portions a day.

It is also possible to concurrently use other pharmaceutical agents for general use, during the prophylaxis and/or treatment of the above-mentioned diseases.

### Examples

The present invention is explained in more detail in the following by referring to Examples, which are not to be construed as limitative.

### (Explanation of measurement device, abbreviation of method and the like)

In Examples 1, 2 and Experimental Examples 1 - 4, each crystal forms was confirmed by powder X-ray diffractometer RINT2500 (manufactured by Rigaku Corporation), thermal analysis equipment TG/DTA6200 (manufactured by Seiko Instruments Inc.) and water vapor sorption apparatus SGA-100 (manufactured by VTI). Powder X-ray diffraction pattern was measured at room temperature using Cu as an X-ray source within the 2θ angle range of 3 - 40°. For thermal analysis, the measurement was performed in a dry nitrogen gas stream (300 mL/min) from 30°C to 350°C at a temperature rise rate of 2°C/min.

In Examples 3, 4 and Experimental Examples 5, 6, each crystal forms was confirmed by powder X-ray diffractometer (manufactured by Bruker) GADDS detector system and thermal analysis equipment (manufactured by Mettler-Toledo) DSC822e system.

Powder X-ray diffraction pattern was measured at room temperature using CuKα as a monochromator within the 2θ angle range of 1.5 - 41.5°.

For thermal analysis, the measurement was performed in a dry nitrogen gas stream (50 mL/min) from 25°C to 300°C at a temperature rise rate of 20°C/min.

For moisture sorption isotherms, when weight variation under each relative humidity condition becomes not more than 0.03% in 5 min, or when 3 hours have passed at each relative humidity, the range of relative humidity being from 0% to 95%, the humidity is increased or decreased at 5% intervals, and the moisture sorption isotherms at each relative humidity is obtained gravimetrically by measuring the mass change of a sample. The "RH" in the preset Examples means "relative humidity".

5-Methyl-2-(piperazin-1-yl)benzenesulfonic acid monohydrate (hereinafter sometimes to be referred to as "caldaret") used in the following Examples was produced according to the method described in patent reference 2, Example 1.

### (Example 1) type-I crystal of 5-methyl-2-(piperazin-1-yl)benzenesulfonic acid anhydrate

5-Methyl-2-(piperazin-1-yl)benzenesulfonic acid monohydrate was heated at 150°C for 2 hr to give a crystal. Since this crystal soon absorbs water in the air and becomes monohydrate, it was dried by an appropriate method during each measurement.

### (Example 2) type-II crystal of 5-methyl-2-(piperazin-1-yl)benzenesulfonic acid anhydrate

N,N-Dimethylformamide (100 mL) was added to 5-methyl-2-(piperazin-1-yl)benzenesulfonic acid monohydrate (3.5 g), and the resulting suspension was stirred as it was at room temperature for 1 hr. The obtained suspension was filtered and dried under reduced pressure at 105°C for 2 hr to give a crystal.

### (Experimental Example 1) Measurement of powder X-ray diffraction

5-Methyl-2-(piperazin-1-yl)benzenesulfonic acid monohydrate was dried under reduced pressure for 2 hr prior to the measurement and used as sample 1 (type-I anhydrate crystal), which was soon (within about 10 minutes after drying under reduced pressure) subjected to the powder X-ray diffraction measurement.

The crystal obtained in Example 2 was used as sample 2 (type-II anhydrate crystal), and subjected to the measurement of powder X-ray diffraction.

Powder X-ray diffraction was measured under the following conditions.
- apparatus :: RINT2500 type powder X-ray equipment (Rigaku Corporation)
- X-ray source :: Cu
- filter :: Ni
- tube voltage :: 40 kV
- tube current :: 300 mA
- divergence slit:: 1/2°
- scattering slit:: 1/2°
- receiving slit:: 0.15 mm
- scanning range :: 3 - 40° 2θ
- angular step :: 0.02°
- sampling time:: 0.50 sec
- sample rotation speed :: 60 rpm
- scan speed:: 0.04°/sec

The powder X-ray diffraction patterns of sample 1 (type-I anhydrate crystal) and sample 2 (type-II anhydrate crystal) are shown in Fig. 1 and Fig. 2, respectively. The diffraction peak values (2θ) of the powder X-ray diffraction pattern of sample 1 (type-I anhydrate crystal) are shown in Table 1, and the diffraction peak values (2θ) of the powder X-ray diffraction pattern of sample 2 (type-II anhydrate crystal) are shown in Table 2.

The powder X-ray diffraction pattern of known product is shown in Fig. 3, and diffraction peak values (2θ) are shown in Table 3.

**Table 1**

| <Diffraction peak values (2θ) of powder X-ray diffraction pattern of type-I crystal> | | |
|---|---|---|
| 6.4 | 16.0 | 21.6 |
| 6.5 | 16.7 | 22.3 |
| 9.6 | 19.5 | 23.3 |
| 13.0 | 19.8 | 25.2 |
| 13.2 | 21.0 | 26.0 |

Of those indicated above, 6.4°, 13.0°, 13.2°, 16.7°, 19.5° and 19.8° are characteristic peaks.

**Table 2**

| <Diffraction peak values (2θ) of powder X-ray diffraction pattern of type-II crystal> | | | | |
|---|---|---|---|---|
| 6.3 | 15.0 | 19.8 | 23.6 | 28.3 |
| 9.8 | 16.0 | 19.8 | 24.0 | 28.7 |
| 11.1 | 16.3 | 21.0 | 24.5 | 28.9 |
| 11.8 | 17.1 | 21.6 | 24.8 | 29.4 |
| 12.8 | 17.6 | 22.3 | 25.0 | 33.0 |
| 14.0 | 18.4 | 22.7 | 27.0 | |
| 14.7 | 19.1 | 23.4 | 27.9 | |

Of those indicated above, 6.3°, 9.8°, 12.8°, 14.0°, 14.7°, 19.1°, 20.5° and 21.0° are characteristic peaks.

**Table 3**

| <Diffraction peak values (2θ) of powder X-ray diffraction pattern of known product> | | |
|---|---|---|
| 6.5 | 14.8 | 20.0 |
| 6.9 | 16.1 | 20.7 |
| 13.0 | 16.7 | 22.3 |
| 13.4 | 18.4 | 25.3 |
| 13.8 | 19.5 | |

As a result of the measurement, it was clarified that sample 1 crystal (type-I anhydrate crystal) and sample 2 crystal (type-II anhydrate crystal) show powder X-ray diffraction patterns different from that of the known product. (Experimental Example 2) Thermal analysis
Monohydrate (5 mg) was placed on an aluminum open-pan, heated once to 150°C, maintained for 30 min and cooled to room temperature. Using the sample as sample 3 (type-I crystal), a thermoanalytical measurement was started.
Sample 2 obtained in Example 2 was weighed (5 mg), placed on an aluminum open-pan, and a thermal analysis was performed. Each measurement was performed under the following conditions.
- apparatus :: Seiko Instruments Inc. TG/DTA6200
- temperature rise rate :: 2°C/min
- atmosphere :: under nitrogen stream 300 mL/min
- measurement temperature :: 30°C - 150°C

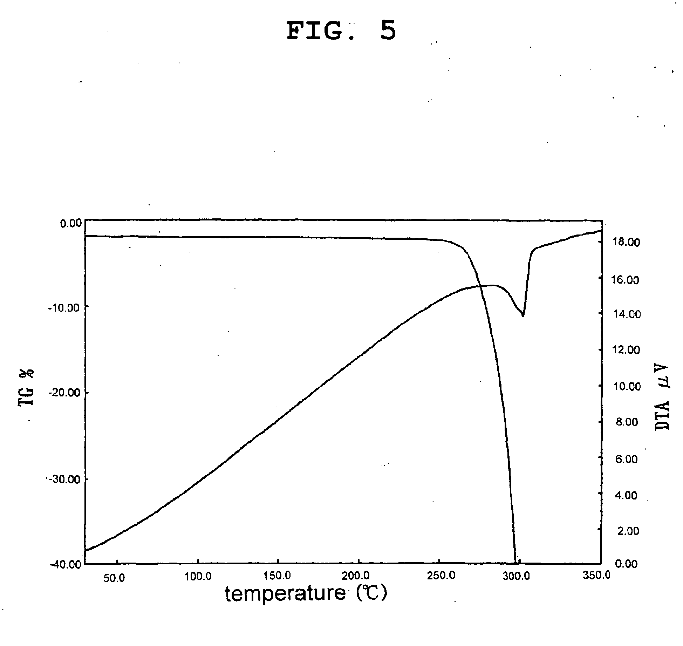

The thermal analysis curves of sample 3 (type-I anhydrate crystal) and sample 2 (type-II anhydrate crystal) are shown in Fig. 4 and Fig. 5, respectively. Both sample 3 (type-I anhydrate crystal) and sample 2 (type-II anhydrate crystal) showed weight decrease and endothermal peaks due to melting (decomposition) at around 270°C.

### (Experimental Example 3) Measurement of infrared spectrum

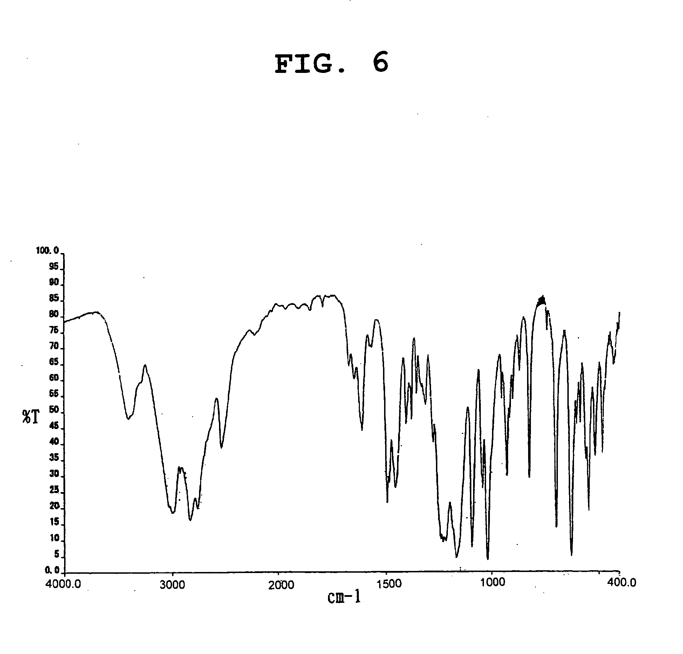

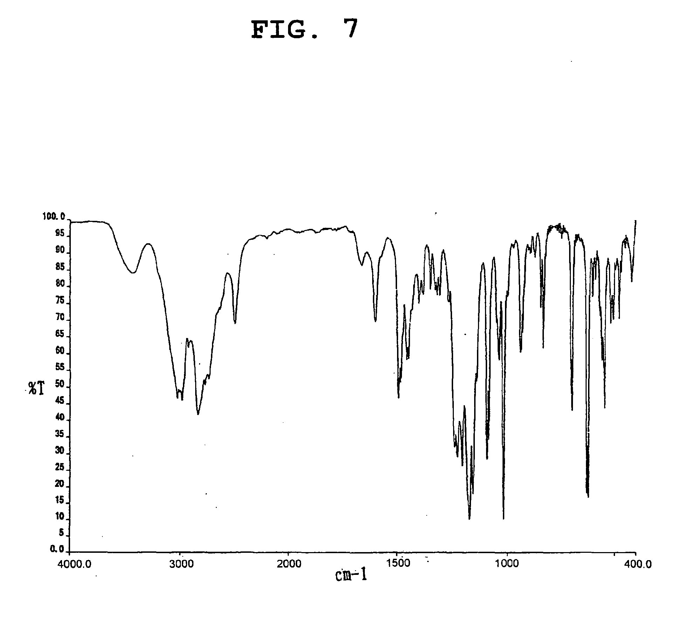

5-Methyl-2-(piperazin-1-yl)benzenesulfonic acid monohydrate was dried under reduced pressure at 150°C for 2 hr prior to the measurement. The sample was used as sample 5 (type-1 anhydrate crystal), and subjected to KCl tablet molding in nitrogen-purged glove box. Thereafter, the prepared KCl tablet was quickly set in a sample booth nitrogen-purged in advance, and infrared spectrum was measured.
The sample 2 (type-II anhydrate crystal) obtained in Example 2 was measured for infrared spectrum by the KCl tablet method.
- apparatus :: Spectrum One (Perkin Elmer)
- measurement range:: 4000 - 400 cm-1
- resolution :: 2.00 cm-1
The infrared spectra of sample 5 (type-I anhydrate crystal) and sample 2 (type-II anhydrate crystal) are shown in Fig. 6 and Fig. 7, respectively.

### (Experimental Example 4) Measurement of moisture sorption at each relative humidity

5-Methyl-2-(piperazin-1-yl)benzenesulfonic acid monohydrate was dried for 60 min by heating in a sample booth at 80°C under nitrogen substitution. The sample was used as sample 7 (type-I anhydrate crystal), and measured for moisture sorption from 5% RH.
The sample 2 (type-II anhydrate crystal) obtained in Example 2 was dried for 60 min by heating in a sample booth at 80°C under nitrogen substitution, and measured for moisture sorption from 5% RH.
The moisture sorption isotherms of sample 7 (type-I anhydrate crystal) and sample 8 (type-II anhydrate crystal) are shown in Fig. 8 and Fig. 9, respectively.
Sample 7 (type-I anhydrate crystal) showed moisture absorption from 10% RH during the process of humidity increase from 5% RH to 95% RH, and a weight increase of about 6.0% at 50% RH (Fig. 8: solid line). Such moisture sorption is assumed to reflect conversion to monohydrate (Calculated 7.03%). After moisture absorption, weight change was not observed even when the humidity was reduced from 95% RH to 5% RH (Fig. 8: dotted line). Therefore, it is clear that hydration water was not eliminated.
The moisture sorption of sample 2 (type-II anhydrate crystal) was about 2.0% in the range of 0 - 50% RH during the process of humidity increase from 5% RH to 95% RH. It is clear that the hygroscopicity was hardly present. A weight increase of about 6.5% was observed around 60% RH (Fig. 9: solid line). After moisture absorption, weight change was not observed even when the humidity was reduced from 95% RH to 5% RH. Therefore, it is clear that hydration water was not eliminated (Fig. 9: dotted line).

From the above-mentioned results, it is clear that the type-I anhydrate crystal of the present invention shows weight change at relative humidity 50% of about 6% and shows less weight change at everyday relative humidity of 40 - 60%.

In addition, it is clear that the type-II anhydrate crystal of the present invention shows moisture sorption at relative humidity 50% of not more than about 2%, and is less hygroscopic and crystallographically stable.

### (Example 3) type-II crystal of 5-methyl-2-(piperazin-1-yl)benzenesulfonic acid monohydrate

5-Methyl-2-(piperazin-1-yl)benzenesulfonic acid monohydrate (25 mg) was heated to 80°C with stirring while gradually adding methanol (about 1 mL). After confirmation of the suspension state of the system, hot filtration was performed to give a saturated solution at 80°C. The saturated solution was gradually cooled (2°C/hr) and the crystal was matured at 4°C for 72 hr to give a white solid.

### (Example 4) type-III crystal of 5-methyl-2-(piperazin-1-yl)benzenesulfonic acid monohydrate

5-Methyl-2-(piperazin-1-yl)benzenesulfonic acid monohydrate (25 mg) was heated to 80°C with stirring while gradually adding formamide (about 0.4 mL). After confirmation of the suspension state of the system, hot filtration was performed to give a saturated solution at 80°C. Water in a 4-fold amount of the volume of formamide was added to the saturated solution to give a yellow-brown solid.

### (Experimental Example 5) Measurement of powder X-ray diffraction

Using the solids obtained in Examples 3, 4 and 5 as sample 8 (type-II monohydrate crystal) and sample 9 (type-III monohydrate crystal), respectively, the powder X-ray diffraction was measured under the following conditions. Measurement conditions
- apparatus :: D8 manufactured by Bruker
- X-ray source :: CuKα
- detector :: GADDS (Hi-STAR)
- measurement range :: 1.5 - 41.5°(2θ)
- measurement temperature :: room temperature
The powder X-ray diffraction patterns of sample 8 (type-II monohydrate crystal) and sample 9 (type-III monohydrate crystal) are shown in Fig. 10 and Fig. 11, respectively.
The characteristic peaks of sample 8 (type-II monohydrate crystal) in terms of diffraction angle represented by 2θ were 7.3, 14.0, 15.5, 19.1, 21.7 and 26.2° (each ±0.2°). The X-ray diffraction pattern was completely different from that of 5-methyl-2-(piperazin-1-yl)benzenesulfonic acid heretofore known, and it was confirmed that the crystal was novel.
The characteristic peaks of sample 9 (type-III monohydrate crystal) in terms of diffraction angle represented by 2θ were 7.4, 14.7, 18.9, 19.4 and 22.3° (each ±0.2°). The X-ray diffraction pattern was completely different from that of 5-methyl-2-(piperazin-1-yl)benzenesulfonic acid heretofore known, and it was confirmed that the crystal was novel.

### (Experimental Example 6) Thermal analysis

Using the solids obtained in Examples 3 and 4 as sample 8 (type-II monohydrate crystal) and sample 9 (type-III monohydrate crystal), respectively, the thermal analysis was performed under the following conditions.

### Measurement conditions

- apparatus :: DSC822e manufactured by Metter-Toledo
- temperature rise rate :: 20°C/min
- atmosphere :: dry nitrogen gas 50 mL/min
- measurement temperature :: 25 - 300°C

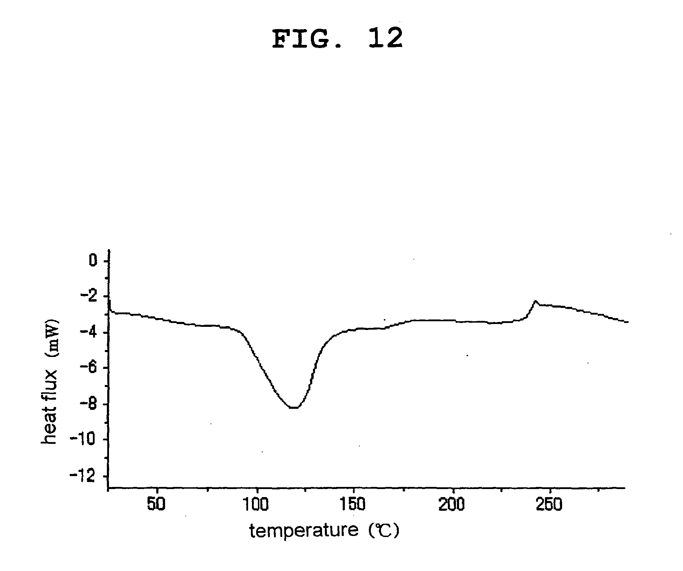

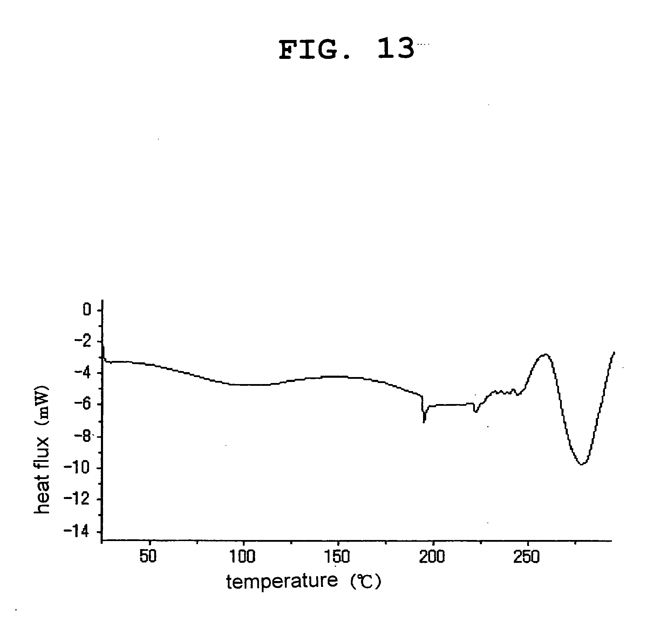

The thermal analysis curves of sample 8 (type-II monohydrate crystal) and sample 9 (type-III monohydrate crystal) are shown in Fig. 12 and Fig. 13, respectively.
Both sample 8 (type-II monohydrate crystal) and sample 9 (type-III monohydrate crystal) did not show a noticeable endothermic and exothermic peaks up to around 80°C, and it was confirmed that the crystals were stable. In addition, the thermal analysis curve was different from those shown in Fig. 4 and Fig. 5, and it was confirmed that the crystals were novel. Accordingly, the novel crystal of the present invention can be stably supplied as an active ingredient drug of pharmaceutical products.

### Industrial Applicability

According to the present invention, type-I crystal and type II crystal, which are novel crystalline polymorphs of 2-(1-piperazinyl)-5-methylbenzenesulfonic acid anhydrate expected to be effective as an active ingredient of prophylactic or therapeutic agents for cardiac diseases, can be provided.

According to the present invention, moreover, type-II crystal and type-III crystal, which are novel crystalline polymorphs of 2-(1-piperazinyl)-5-methylbenzenesulfonic acid monohydrate expected to be effective as an active ingredient of prophylactic or therapeutic agents for cardiac diseases, can be provided.

This application is based on a patent application No. 2006-200072 filed in Japan, the contents of which are incorporated in full herein by this reference.

## Claims

1. 5-Methyl-2-(piperazin-1-yl)benzenesulfonic acid anhydrate **characterized by** the following (a) and (b):
(a) showing a diffraction peak at at least one Bragg angle (2θ) selected from 6.4°, 13.0°, 16.7°, 19.5° and 19.8° (each ±0.2°) in a powder X-ray diffraction pattern,
(b) showing no diffraction peak at any of Bragg angles (2θ) 14.0°, 14.7° and 19.1° (each ±0.2°) in a powder X-ray diffraction pattern.

2. The 5-methyl-2-(piperazin-1-yl)benzenesulfonic acid anhydrate of claim 1, showing diffraction peaks at Bragg angles (2θ) of 16.7° and 19.5° (each ±0.2°) in a powder X-ray diffraction pattern.

3. The 5-methyl-2-(piperazin-1-yl)benzenesulfonic acid anhydrate of claim 1, showing diffraction peaks at Bragg angles (2θ) of 6.4°, 16.7° and 19.5° (each ±0.2°) in a powder X-ray diffraction pattern.

4. The 5-methyl-2-(piperazin-1-yl)benzenesulfonic acid anhydrate of claim 1, showing diffraction peaks at Bragg angles (2θ) of 6.4°, 13.0°, 16.7° and 19.5° (each ±0.2°) in a powder X-ray diffraction pattern.

5. The 5-methyl-2-(piperazin-1-yl)benzenesulfonic acid anhydrate of claim 1, showing diffraction peaks at Bragg angles (2θ) of 6.4°, 13.0°, 16.7°, 19.5° and 19.8° (each ±0.2°) in a powder X-ray diffraction pattern.

6. 5-Methyl-2-(piperazin-1-yl)benzenesulfonic acid anhydrate having substantially the same powder X-ray diffraction pattern as that showing Bragg angles (2θ) in the powder X-ray diffraction pattern of Fig. 1.

7. 5-Methyl-2-(piperazin-1-yl)benzenesulfonic acid anhydrate showing substantially the same moisture sorption isotherms as that of Fig. 8.

8. The 5-methyl-2-(piperazin-1-yl)benzenesulfonic acid anhydrate of any of claims 1 to 6, which shows substantially the same moisture sorption isotherms as that of Fig. 8.

9. 5-Methyl-2-(piperazin-1-yl)benzenesulfonic acid anhydrate showing moisture sorption at relative humidity 50% of not less than about 6%.

10. The 5-methyl-2-(piperazin-1-yl)benzenesulfonic acid anhydrate of any of claims 1 to 6, showing moisture sorption at relative humidity 50% of not less than about 6%.

11. 5-Methyl-2-(piperazin-1-yl)benzenesulfonic acid anhydrate **characterized by** the following (c) and (d):
(c) showing a diffraction peak at at least one Bragg angle (2θ) selected from 6.3°, 9.8°, 12.8°, 14.0°, 14.7° and 19.1° (each ±0.2°) in a powder X-ray diffraction pattern,
(d) showing no diffraction peak at any of Bragg angles (2θ) of 16.7° and 19.5° (each ±0.2°) in a powder X-ray diffraction pattern.

12. The 5-methyl-2-(piperazin-1-yl)benzenesulfonic acid anhydrate of claim 11, showing diffraction peaks at Bragg angles (2θ) of 14.0° and 14.7° (each ±0.2°) in a powder X-ray diffraction pattern.

13. The 5-methyl-2-(piperazin-1-yl)benzenesulfonic acid anhydrate of claim 11, showing diffraction peaks at Bragg angles (2θ) of 14.0°, 14.7° and 19.1° (each ±0.2°) in a powder X-ray diffraction pattern.

14. The 5-methyl-2-(piperazin-1-yl)benzenesulfonic acid anhydrate of claim 11, showing diffraction peaks at Bragg angles (2θ) of 6.3°, 14.0°, 14.7° and 19.1° (each ±0.2°) in a powder X-ray diffraction pattern.

15. The 5-methyl-2-(piperazin-1-yl)benzenesulfonic acid anhydrate of claim 11, showing diffraction peaks at Bragg angles (2θ) of 6.3°, 9.8°, 14.0°, 14.7° and 19.1° (each ±0.2°) in a powder X-ray diffraction pattern.

16. 5-Methyl-2-(piperazin-1-yl)benzenesulfonic acid anhydrate having substantially the same powder X-ray diffraction pattern as that showing Bragg angles (2θ) in the powder X-ray diffraction pattern of Fig. 2.

17. 5-Methyl-2-(piperazin-1-yl)benzenesulfonic acid anhydrate showing substantially the same moisture sorption isotherms as that of Fig. 9.

18. The 5-methyl-2-(piperazin-1-yl)benzenesulfonic acid anhydrate of any of claims 11 to 16, showing substantially the same moisture sorption isotherms as that of Fig. 9.

19. 5-Methyl-2-(piperazin-1-yl)benzenesulfonic acid anhydrate showing moisture sorption at relative humidity 50% of not more than about 2%.

20. The 5-methyl-2-(piperazin-1-yl)benzenesulfonic acid anhydrate of any of claims 11 to 20, showing moisture sorption at relative humidity 50% of not more than about 2%.

21. 5-Methyl-2-(piperazin-1-yl)benzenesulfonic acid monohydrate **characterized by** the following (e) and (f):
(e) showing a diffraction peak at at least one Bragg angle (2θ) selected from 7.3°, 14.0°, 15.5°, 19.1°, 21.7° and 26.2° (each ±0.2°) in a powder X-ray diffraction pattern,
(f) showing no diffraction peak at any of Bragg angles (2θ) of 13.3°, 25.4° and 27.6° (each ±0.2°) in a powder X-ray diffraction pattern.

22. 5-Methyl-2-(piperazin-1-yl)benzenesulfonic acid monohydrate showing substantially the same powder X-ray diffraction pattern as that showing Bragg angles (2θ) in the powder X-ray diffraction pattern of Fig. 10.

23. 5-Methyl-2-(piperazin-1-yl)benzenesulfonic acid monohydrate **characterized by** the following (g) and (h):
(g) showing a diffraction peak at at least one Bragg angle (2θ) selected from 7.4°, 14.7°, 18.9°, 19.4° and 22.3° (each ±0.2°) in a powder X-ray diffraction pattern,
(h) showing no diffraction peak at any of Bragg angles (2θ) of 13.3°, 13.7°, 15.6°, 25.4° and 27.6° (each ±0.2°) in a powder X-ray diffraction pattern.

24. 5-Methyl-2-(piperazin-1-yl)benzenesulfonic acid monohydrate having substantially the same powder X-ray diffraction pattern as that showing Bragg angles (2θ) in the powder X-ray diffraction pattern of Fig. 11.

25. A drug substance consisting of the 5-methyl-2-(piperazin-1-yl)benzenesulfonic acid anhydrate of any of claims 1 to 20.

26. A drug substance consisting of the 5-methyl-2-(piperazin-1-yl)benzenesulfonic acid monohydrate of any of claims 21 to 24.

27. A pharmaceutical composition comprising the 5-methyl-2-(piperazin-1-yl)benzenesulfonic acid anhydrate of any of claims 1 to 20 as an active ingredient.

28. A pharmaceutical composition comprising the 5-methyl-2-(piperazin-1-yl)benzenesulfonic acid monohydrate of any of claims 21 to 24 as an active ingredient.

29. The pharmaceutical composition of claim 27 or 28, which is used for the prophylaxis and/or treatment of an ischemic cardiac disease or an ischemic circulatory disorder.

30. The pharmaceutical composition of claim 27 or 28, which is used for the prophylaxis and/or treatment of muscle infarction, angina pectoris, cardiac failure, hypertension, arrhythmia, cardiomyopathy, diastolic disorder, nerve system disorder, cerebrovascular disorder, ischemic cerebrovascular disorder, or cardiac failure or arrhythmia in ischemic cardiac disease derived from diabetes.

31. The pharmaceutical composition of claim 27 or 28, which is used for the prophylaxis and/or treatment of a circulatory disorder by administration to a patient undergoing percutaneous coronary intervention.

32. A method of producing a type-I crystal of the 5-methyl-2-(piperazin-1-yl)benzenesulfonic acid anhydrate of any of claims 1 to 10, which comprises crystallizing or washing, in a suspension state, 5-methyl-2-(piperazin-1-yl)benzenesulfonic acid solvate using a solvent containing water, and drying the obtained crystal.

33. A method of producing a type-II crystal of the 5-methyl-2-(piperazin-1-yl)benzenesulfonic acid anhydrate of any of claims 11 to 20, which comprises crystallizing or washing, in a suspension state, 5-methyl-2-(piperazin-1-yl)benzenesulfonic acid solvate using a solvent free of water.
